# EUROPÄISCHE PATENTSCHRIFT

(11) **EP 3 378 928 B1**
(45) Veröffentlichungstag und Bekanntmachung des Hinweises auf die Patenterteilung: **19.04.2023**
(21) Anmeldenummer: 18159932.5
(22) Anmeldetag: 05.03.2018
(51) Int. Cl.: F24F 6/18, F24F 6/00, C12M 1/02, F24F 6/02, F24F 11/00, C12M 1/00, C12M 1/34, C12M 1/36

(54) **VERFAHREN ZUM BETRIEB EINES BEFEUCHTUNGSMODULS, BEFEUCHTUNGSMODUL UND INKUBATOR ODER KLIMASCHRANK MIT BEFEUCHTUNGSMODUL**
HUMIDIFYING MODULE AND INCUBATOR OR AIR CONDITIONED CABINET WITH HUMIDIFYING MODUL AND METHOD FOR OPERATING A HUMIDIFYING MODULE
PROCÉDÉ DE FONCTIONNEMENT UN MODULE D'HUMIDIFICATION, MODULE D'HUMIDIFICATION ET INCUBATEUR OU ARMOIRE CLIMATISÉE POURVUS DE MODULE D'HUMIDIFICATION

(30) Priorität: 20.03.2017 DE 102017105892
(43) Veröffentlichungstag der Anmeldung: 26.09.2018
(73) Patentinhaber: Binder GmbH, 78532 Tuttlingen (DE)
(72) Erfinder: Buschle, Jochen, 78532 Tuttlingen (DE)
(74) Vertreter: Westphal, Mussgnug & Partner Patentanwälte mbB

(56) Entgegenhaltungen:
- EP-A1- 0 340 341
- WO-A1-2012/093326
- WO-A1-2015/172882
- DE-A1-102014 110 965

## Beschreibung

Mit Hilfe von Inkubatoren oder Klimaschränken ist es möglich, definierte klimatische Bedingungen in einem innerhalb des Inkubators oder Klimaschranks befindlichen Prüfraum herzustellen, insbesondere eine vorgegebene Temperatur und Luftfeuchtigkeit. Die Luftfeuchtigkeit ist in vielen Anwendungsfällen, beispielsweise beim Bebrüten von Zellen, wo teilweise Luftfeuchtigkeiten >90% r.F. eingestellt und aufrecht erhalten werden müssen eine wichtige Kenngröße. Dennoch wird sie bislang in vielen Fällen einfach dadurch geregelt, dass ein Wasserreservoir, z.B. eine Schale mit Wasser, mit in den Inkubator oder Klimaschrank gestellt wird, das dann durch Verdunstung bei den jeweils im Inkubator oder Klimaschrank herrschenden Temperaturen in die Luft im Innenraum des Inkubators oder Klimaschranks übergeht.

Vor allem bei Türöffnungen wird das Klima im Inkubator oder Klimaschrank gestört, was dann insbesondere mit sich bringt, dass die Luftfeuchtigkeit unter den geforderten Wert fällt. Um Einflüsse solcher Abweichungen von den Sollwerten möglichst gering zu halten, ist eine kurze Feuchteerholzeit wesentlich, was insbesondere erfordert, eine möglichst kontinuierliche Befeuchtung sicherzustellen.

Aus der DE 10 2014 110 965 A1 ist ein Dampfgenerator für ein Gargerät mit einem Wasserkessel, mindestens einer Heizvorrichtung und einem Dampfauslass bekannt, bei dem eine Einspritzdüse vorgesehen ist, mit der Wasser auf eine oberhalb eines im Wasserspiegels angeordnete Heizvorrichtung gesprüht werden kann. Weiter offenbart diese Druckschrift ein Verfahren zum Betreiben eines Dampfgenerators, bei dem auf ein Dampf-Anforderungssignal hin auf einen Abschnitt dieses Heizelements Wasser aufgebracht wird, so dass ein Dampfstoß entsteht und ein Gargerät mit einem derartigen Dampfgenerator.

Aus der WO 2015/172882 A1 ist ein modulares Inkubatorsystem bekannt, bei dem insbesondere ein Temperatursensor zur Überwachung der Temperatur im Innenraum des Inkubators vorgesehen ist und das darüber hinaus einen Anschluss zur Versorgung mit Dampf aufweist.

Die Aufgabe der Erfindung besteht darin, ein Verfahren zum Betrieb eines Befeuchtungsmoduls, wobei das Befeuchtungsmodul insbesondere für einen Inkubator oder Klimaschrank geeignet ist, ein Befeuchtungsmodul zur Durchführung des Verfahrens, das insbesondere für einen Inkubator oder Klimaschrank geeignet ist, und einen Inkubator oder Klimaschrank mit einem solchen Befeuchtungsmodul bereitzustellen, mit dem auf einfache und effiziente Weise eine möglichst kontinuierliche Befeuchtung gesichert werden kann.

Diese Aufgabe wird gelöst durch ein Verfahren zum Betrieb eines Befeuchtungsmoduls mit den Merkmalen des Patentanspruchs 1, durch ein Befeuchtungsmodul zur Durchführung des Verfahrens mit den Merkmalen des Patentanspruchs 4 und durch einen Inkubator oder Klima-schrank mit Befeuchtungsmodul mit den Merkmalen des Anspruchs 10. Vorteilhafte Weiterbildungen der Erfindung sind Gegenstand der jeweiligen abhängigen Ansprüche.

Mit dem erfindungsgemäßen Verfahren wird ein Befeuchtungsmodul, das insbesondere für einen Inkubator oder Klimaschrank geeignet ist, betrieben, wobei das Befeuchtungsmodul einen Behälter, der einen Wassereinlass und einen Dampfauslass hat, eine Heizvorrichtung, die im Innenraum des Behälters angeordnet ist, und einen Temperatursensor und eine Steuerschaltung zur Steuerung oder Regelung einer Wasserzufuhr durch den Wassereinlass und/oder zur Steuerung oder Regelung einer Heizleistung der Heizvorrichtung aufweist.

Erfindungswesentlich ist, dass der Behälter maximal soweit mit Wasser gefüllt wird, dass ein Dampfvolumen verbleibt, in dem sich beim Heizen mit der Heizvorrichtung, z.B. einer elektrischen Heizpatrone, erzeugter Wasserdampf befindet, dass ein Teil der Heizvorrichtung aus dem Wasser herausragt und in das Dampfvolumen hineinragt -was auch der Fall ist, wenn ein Teil der Heizvorrichtung das Dampfvolumen durchquert-, so dass der Wasserdampf überhitzt werden kann, und dass der Temperatursensor so angeordnet ist, dass er direkt oder indirekt die Temperatur des Wasserdampfs misst, wobei die gemessene Temperatur als Regelungsparameter von der Steuerschaltung verwendet wird. Eine direkte Messung liegt dann von, wenn die Messstelle des Temperatursensor innerhalb des Dampfvolumens liegt; es sind aber auch indirekte Messungen möglich, bei denen die Messstelle des Temperatursensors an der Behälterwand im Bereiche des Dampfvolumens angeordnet ist oder im Inneren der Heizvorrichtung angeordnet ist.

Zur Erzeugung von Dampf wird das Wasser durch Beheizen mit der Heizvorrichtung erwärmt, so dass Dampf in das Dampfvolumen übertritt. Dort kommt es zur weiteren Erwärmung des Dampfes, der überhitzt wird und dabei weiter in Richtung auf den Dampfauslass aufsteigt, durch den er dann zur Befeuchtung in den Innenraum des Inkubators oder Klimaschranks geleitet werden kann, wenn dies gewünscht ist. Neben der Tatsache, dass diese Betriebsweise es ermöglicht, ein druckloses System zu betreiben, was einen einfachen und kostengünstigen Aufbau ermöglicht und zudem die Zulassung erleichtert, wird durch eine Überhitzung des Dampfes im Dampfvolumen auch eine Sterilisierung erreicht, so dass das Risiko, dass Fremdorganismen in den Inkubator oder Klimaschrank gelangen, deutlich reduziert wird.

Die direkte oder indirekte Temperaturüberwachung der Dampftemperatur wird erfindungsgemäß zudem als Regelparameter eingesetzt. Einerseits kann auf diese Weise das Nachfüllen von Wasser gesteuert werden, indem beim Erreichen oder Überschreiten eines Schwellenwerts der von dem Temperatursensor gemessenen Temperatur des Wasserdampfs durch die Steuerschaltung Wasser über den Wassereinlass in den Behälter eingelassen wird. Bei andauerndem Betrieb des Befeuchtungsmoduls sinkt der Wasserspiegel, da immer mehr Wasser verdampft wird. Dementsprechend wird der entstandene Dampf immer weiter überhitzt, weil das Dampfvolumen sich mit sinkendem Wasserspiegel vergrößert und somit ein immer größerer Teil der Heizvorrichtung aus dem Wasser hervorragt. Bei einer gegebenen Heizleistung ist somit aus der Dampftemperatur, die erreicht wird, ein Rückschluss auf den aktuellen Wasserfüllstand möglich, so dass ein rechtzeitiges automatisches Nachfüllen und somit ein möglichst kontinuierlicher Dauerbetrieb ermöglicht wird.

Es ist aber auch möglich, die Belastung des Befeuchtungsmoduls zu reduzieren, wenn das Befeuchtungsmodul in einem Bereitschaftsmodus betrieben wird, wenn es keine Feuchtigkeit in Form von Wasserdampf abgeben muss. In diesem Bereitschaftsmodus wird dann die Heizleistung des Heizelements im Bereitschaftsmodus durch die Steuerschaltung unter Verwendung der vom Temperatursensor gemessenen Temperatur so geregelt, dass die Temperatur unterhalb der Siedetemperatur bleibt.

Das erfindungsgemäße Befeuchtungsmodul, das insbesondere zur Durchführung eines solchen Verfahrens für einen Inkubator oder Klimaschrank geeignet ist, hat einen Behälter, der einen Wassereinlass und einen Dampfauslass aufweist, eine Heizvorrichtung, die so im Innenraum des Behälters angeordnet ist, dass sie auch bei maximaler Befüllung des Behälters mit Wasser teilweise aus dem Wasser herausragt, einen Temperatursensor und eine Steuerschaltung, die zur Steuerung oder Regelung einer Wasserzufuhr durch den Wassereinlass und/oder zur Steuerung oder Regelung einer Heizleistung der Heizvorrichtung basierend auf Messwerten des Temperatursensors eingerichtet ist.

Angemerkt sei, dass eine maximale Befüllung des Behälters bereits durch die Position des Dampfauslasses vorgegeben ist, der zwingend oberhalb des Wasserspiegels liegen muss, damit das Wasser nicht herausläuft. Implizit ist damit der Behälterinneraum immer in ein Dampfvolumen und ein Flüssigkeitsvolumen unterteilt, wobei das Flüssigkeitsvolumen unten und das Dampfvolumen oben ist. Mit zunehmender Verdampfung verschiebt sich die Grenze zwischen diesen beiden Volumina.

Dies gilt insbesondere bei seitlicher Anordnung des Dampfauslasses. Selbst in Fällen, in denen keine Markierung zur Vorgabe eines maximalen Wasserfüllstands vorhanden ist, ist somit eine maximale Befüllung des Behälters durch den Aufbau des Behälters vorgegeben.

In einer ersten Ausführungsform ist der Temperatursensor am Außenmantel des Behälters oberhalb des maximalen Wasserfüllstands auf Höhe des Dampfvolumens angeordnet ist. Alternativ dazu kann der Temperatursensor innerhalb des Dampfvolumens oder innerhalb der Heizvorrichtung angeordnet sein.

Besonders bevorzugt ist es, wenn die Steuerschaltung dazu eingerichtet ist, beim Erreichen oder Überschreiten eines Schwellenwerts der von dem Temperatursensor gemessenen Temperatur des Wasserdampfs Wasser über den Wassereinlass in den Behälter einzulassen.

Alternativ oder zusätzlich kann die Steuerschaltung bevorzugt dazu eingerichtet sein, das Befeuchtungsmodul in einem Bereitschaftsmodus zu betreiben, wenn es keine Feuchtigkeit in Form von Wasserdampf abgeben muss, und ferner eingerichtet sein, die Heizleistung des Heizelements im Bereitschaftsmodus durch die Steuerschaltung unter Verwendung der vom Temperatursensor gemessenen Temperatur so regeln, dass die Temperatur unterhalb der Siedetemperatur bleibt.

Der erfindungsgemäße Inkubator oder Klimaschrank zeichnet sich dadurch aus, dass er ein erfindungsgemäßes Befeuchtungsmodul aufweist.

Die Erfindung wird nachfolgend anhand von Figuren, die Ausführungsbeispiele zeigen, näher erläutert. Es zeigt:
Fig. 1: Ein erstes Befeuchtungsmodul, insbesondere für einen Inkubator oder Klimaschrank,
Fig. 2: ein zweites Befeuchtungsmodul, insbesondere für einen Inkubator oder Klimaschrank, und
Fig. 3: ein drittes Befeuchtungsmodul, insbesondere für einen Inkubator oder Klimaschrank.

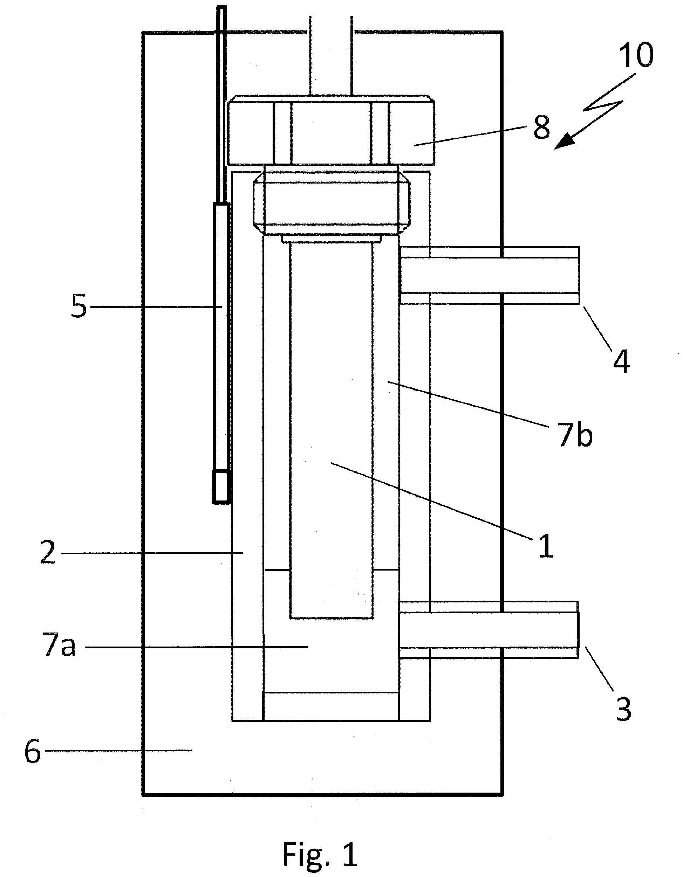

Figur 1 zeigt ein Befeuchtungsmodul 10, das insbesondere für einen Inkubator oder Klimaschrank geeignet ist. Das Befeuchtungsmodul 10 weist einen Behälter 2 auf, dessen Innenraum sich in ein mit Wasser befülltes Flüssigkeitsvolumen 7a, das aus physikalischen Gründen unten liegt, und ein darüber liegendes Dampfvolumen 7b aufteilt. Im Bereich des Flüssigkeitsvolumens 7a ist ein seitlicher Wassereinlass 3 vorgesehen, während im Bereich des Dampfvolumens 7b ein Dampfauslass 4 vorhanden ist, der zugleich einen maximalen Wasserfüllstand definiert, so dass im Behälter 2 immer ein Dampfvolumen 7b verbleibt. Der Behälter 2 ist mit einer Isolationsschicht 6 thermisch isoliert, um einen energieeffizienteren Betrieb des Befeuchtungsmoduls 10 zu ermöglichen.

Ebenfalls im Behälter 2 angeordnet ist eine Heizvorrichtung 1, welche beispielsweise als selbstbegrenzende PTC-Heizpatrone ausgeführt sein kann. Dabei ist die Heizvorrichtung 1 nicht vollständig von Wasser bedeckt, sondern ragt aus dem Wasser heraus in das Dampfvolumen 7b hinein. Im gezeigten Ausführungsbeispiel ist die Heizvorrichtung 1 an einem Deckel 8 des Behälters angeordnet und erstreckt sich somit durch das gesamte Dampfvolumen 7b hindurch.

Dementsprechend wird beim Betrieb des Befeuchtungsmoduls 10 Wasser mit der Heizvorrichtung 1 erwärmt und steigt als Dampf in das Dampfvolumen 7b auf. Weil in diesem Dampfvolumen 7b weiter durch die Heizvorrichtung 1 Heizleistung bereitgestellt wird, kommt es zu einer Überhitzung des Dampfes, der dann bei Bedarf durch den im oberen Bereich des Behälters vorhandenen Dampfauslass 4 in den nicht dargestellten Innenraum des Inkubators übertreten kann und dort für die gewünschte Befeuchtung sorgt.

Im Bereich des Dampfvolumens 7b ist an einer Außenwand des Behälters anliegend ein Temperatursensor 5, beispielsweise ein PT-100 Element, angeordnet, mit dem hier indirekt die Temperatur des Überhitzten Dampfes nachgewiesen wird. Die Messwerte des Temperatursensors 5 werden an eine in Figur 1 nicht dargestellte Steuerschaltung weitergeleitet, die insbesondere dann, wenn die gemessene Temperatur einen Schwellenwert überschreitet, das Nachfüllen von Wasser durch den Wassereinlass 3 veranlasst.

Wenn gerade keine Feuchtigkeit dem Inkubator oder Klimaschrank zugeführt werden muss, wird durch die Steuerschaltung das Befeuchtungsmodul 10 in einem Bereitschaftsmodus betrieben, in dem die Heizleistung der Heizvorrichtung 1 so weit reduziert wird, dass die an dem Temperatursensor 5 gemessene Temperatur unterhalb der Siedetemperatur liegt und durch Verwendung der Daten des Temperatursensors 5 als Regelgröße knapp unterhalb der Siedetemperatur stabilisiert werden, so dass bei einem erneuten Feuchtigkeitsbedarf im Innenraum des Inkubators oder Klimaschranks durch Erhöhung der Heizleistung der Heizvorrichtung 1 schnell wieder Dampf bereitgestellt werden kann, gleichzeitig aber solange das nicht der Fall ist das Befeuchtungsmodul 10 in einem weniger stark belastenden Betriebsmodus betrieben wird.

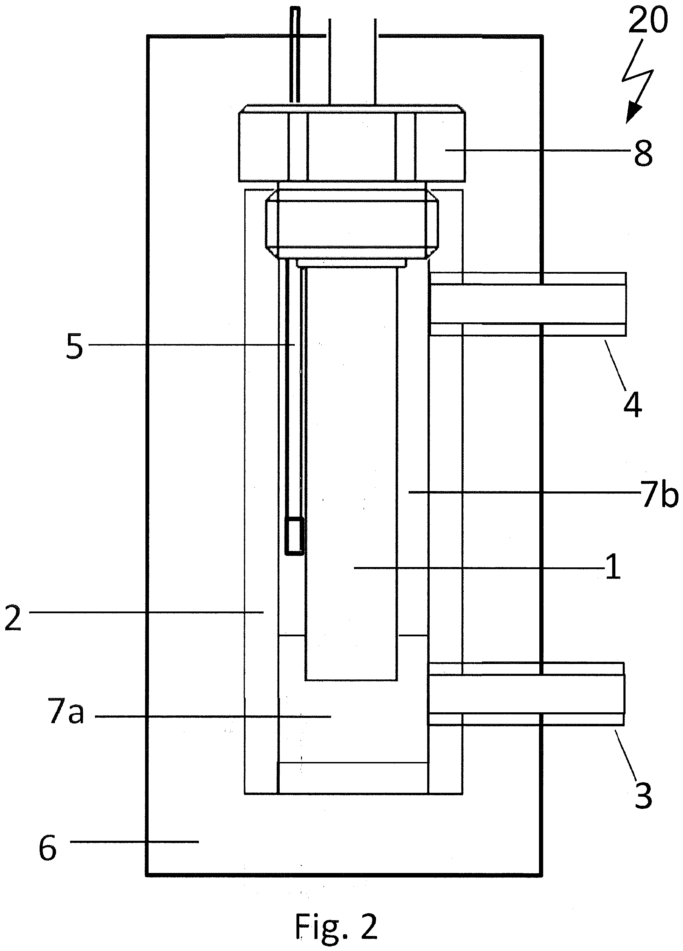

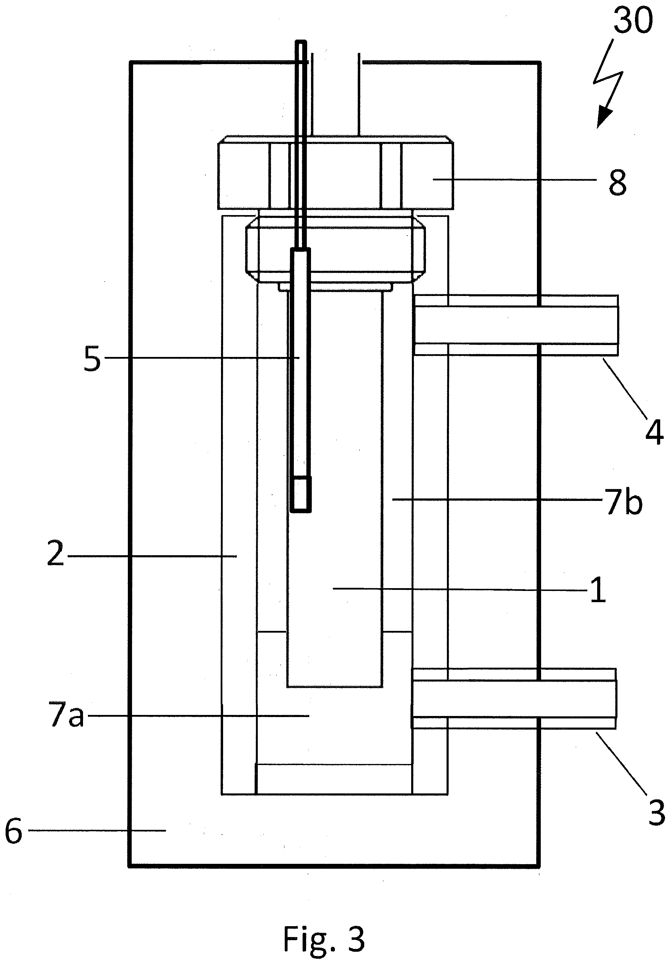

Die Befeuchtungsmodule 20 bzw. 30, die in den Figuren 2 und 3 dargestellt sind, weisen dieselben Bauteile auf, die daher auch mit denselben Bezugszeichen gekennzeichnet sind. Der Unterschied zwischen den Befeuchtungsmodulen 10,20,30 besteht lediglich in der Anordnung des Temperatursensors 5. In Figur 2 ist dieser direkt im Dampfvolumen 7b angeordnet, was eine direkte Messung mit sich bringt. In Figur 3 ist er in das Innere der Heizvorrichtung 1 aufgenommen.

### Bezugszeichenliste

- 1: Heizvorrichtung
- 2: Behälter
- 3: Wassereinlass
- 4: Dampfauslass
- 5: Temperatursensor
- 6: Isolationsschicht
- 7a: Flüssigkeitsvolumen
- 7b: Dampfvolumen
- 8: Deckel
- 10,20,30: Befeuchtungsmodul

## Patentansprüche

1. Verfahren zum Betrieb eines Befeuchtungsmoduls (10,20,30), wobei das Befeuchtungsmodul(10,20,30)
einen Behälter (2), der einen Wassereinlass (3) und einen Dampfauslass (4) hat,
eine Heizvorrichtung (1), die im Innenraum des Behälters (2) angeordnet ist, und
einen Temperatursensor (5) und eine Steuerschaltung zur Steuerung oder Regelung einer Wasserzufuhr durch den Wassereinlass (3) und/oder zur Steuerung oder Regelung einer Heizleistung der Heizvorrichtung (1) aufweist
**dadurch gekennzeichnet, dass**
der Behälter (2) maximal so weit mit Wasser gefüllt wird, dass ein Dampfvolumen (7b) verbleibt, in dem sich beim Heizen mit der Heizvorrichtung (1) erzeugter Wasserdampf befindet,
dass ein Teil der Heizvorrichtung (1) aus dem Wasser herausragt und in das Dampfvolumen (7b) hineinragt, so dass der Wasserdampf überhitzt werden kann, und
dass der Temperatursensor (5) so angeordnet ist, dass er direkt oder indirekt die Temperatur des Wasserdampfs misst, wobei die gemessene Temperatur als Regelungsparameter von der Steuerschaltung verwendet wird.

2. Verfahren nach Anspruch 1,
**dadurch gekennzeichnet, dass** beim Erreichen oder Überschreiten eines Schwellenwerts der von dem Temperatursensor (5) gemessenen Temperatur des Wasserdampfs durch die Steuerschaltung Wasser über den Wassereinlass (3) in den Behälter (2) eingelassen wird.

3. Verfahren nach Anspruch 1 oder 2,
**dadurch gekennzeichnet, dass** das Befeuchtungsmodul (10,20,30) in einem Bereitschaftsmodus betrieben wird, wenn es keine Feuchtigkeit in Form von Wasserdampf abgeben muss, und dass die Heizleistung des Heizelements (1) im Bereitschaftsmodus durch die Steuerschaltung unter Verwendung der vom Temperatursensor (5) gemessenen Temperatur so geregelt wird, dass die Temperatur unterhalb der Siedetemperatur bleibt.

4. Befeuchtungsmodul (10,20,30) zur Durchführung eines Verfahrens nach einem der Ansprüche 1 bis 3 mit einem Behälter (2), der einen Wassereinlass (3) und einen Dampfauslass (4) aufweist, mit einer Heizvorrichtung (1), die so im Innenraum des Behälters (2) angeordnet ist, dass sie auch bei maximaler Befüllung des Behälters (2) mit Wasser teilweise aus dem Wasser herausragt, mit einem Temperatursensor (5) und mit einer Steuerschaltung die zur Steuerung oder Regelung einer Wasserzufuhr durch den Wassereinlass (3) und/oder zur Steuerung oder Regelung einer Heizleistung der Heizvorrichtung (1) basierend auf Messwerten des Temperatursensors (5) eingerichtet ist.

5. Befeuchtungsmodul (10,20,30) nach Anspruch 4,
**dadurch gekennzeichnet, dass** der Temperatursensor (5) am Außenmantel des Behälters (2) oberhalb des maximalen Wasserfüllstands auf Höhe des Dampfvolumens (7b) angeordnet ist.

6. Befeuchtungsmodul (10,20,30) nach Anspruch 4,
**dadurch gekennzeichnet, dass** der Temperatursensor (5) innerhalb des Dampfvolumens (7b)angeordnet ist.

7. Befeuchtungsmodul (10,20,30) nach Anspruch 4,
**dadurch gekennzeichnet, dass** der Temperatursensor (5) innerhalb der Heizvorrichtung (1) angeordnet ist.

8. Befeuchtungsmodul (10,20,30) nach einem der Ansprüche 4 bis 7,
**dadurch gekennzeichnet, dass** die Steuerschaltung dazu eingerichtet ist, beim Erreichen oder Überschreiten eines Schwellenwerts der von dem Temperatursensor (5) gemessenen Temperatur des Wasserdampfs Wasser über den Wassereinlass (3) in den Behälter (2) einzulassen.

9. Befeuchtungsmodul (10,20,30) nach einem der Ansprüche 4 bis 8,
**dadurch gekennzeichnet, dass** die Steuerschaltung dazu eingerichtet ist, das Befeuchtungsmodul (10,20,30) in einem Bereitschaftsmodus zu betreiben, wenn es keine Feuchtigkeit in Form von Wasserdampf abgeben muss, und ferner eingerichtet ist, die Heizleistung des Heizelements (1) im Bereitschaftsmodus durch die Steuerschaltung unter Verwendung der vom Temperatursensor (5) gemessenen Temperatur so regeln, dass die Temperatur unterhalb der Siedetemperatur bleibt.

10. Inkubator oder Klimaschrank mit einem Befeuchtungsmodul (10,20,30),
**dadurch gekennzeichnet, dass** das Befeuchtungsmodul (10,20,30) ein Befeuchtungsmodul (10,20, 30) nach einem der Ansprüche 4 bis 9 ist.

## Claims

1. Method for the operation of a humidification module (10, 20, 30),
wherein the humidification module (10, 20, 30) comprises
a container (2), which has a water inlet (3) and a steam outlet (4),
a heating device (1), which is arranged in the inner chamber of the container (2), and a temperature sensor (5) and a control circuit for the control or regulation of a water supply through the water inlet (3) and/or for the control or regulation of a heat output of the heating device (1),
**characterized in that**
the container (2) is filled with water to the maximum extent at which a steam volume (7b) remains which contains water vapor produced by heating with the heating device (1),
**in that** part of the heating device (1) extends out of the water and extends into the steam volume (7b) such that the water vapor can be superheated, and
**in that** the temperature sensor (5) is arranged such that it directly or indirectly measures the temperature of the water vapor, wherein the measured temperature is used by the control circuit as a regulation parameter.

2. Method in accordance with claim 1,
**characterized in that**, when a threshold value of the water vapor temperature measured by the temperature sensor (5) is reached or surpassed, water is released into the container (2) via the water inlet (3) by means of the control circuit.

3. Method in accordance with claim 1 or 2,
**characterized in that** the humidification module (10, 20, 30) is operated in a standby mode when it is not required to generate humidity in the form of water vapor, and **in that** the heat output of the heating device (1) in the standby mode is adjusted by means of the control circuit, based on the temperature measured by the temperature sensor (5), such that the temperature remains below the boiling temperature.

4. Humidification module (10, 20, 30) for carrying out any of the methods in accordance with any of claims 1 to 3, with a container (2), which comprises a water inlet (3) and a steam outlet (4), with a heating device (1), which is arranged in the inner chamber of the container (2) such that, even when the container (2) is filled to its maximum with water, it still partially extends out of the water, with a temperature sensor (5), and with a control circuit, which is implemented to control or adjust a water supply via the water inlet (3), and/or to control or adjust a heat output of the heating device (1), based on the measured values of the temperature sensor (5).

5. Humidification module (10, 20, 30) in accordance with claim 4,
**characterized in that** the temperature sensor (5) is arranged on the outer casing of the container (2) above the maximum water filling level, at the height of the steam volume (7b).

6. Humidification module (10, 20, 30) in accordance with claim 4,
**characterized in that** the temperature sensor (5) is arranged inside of the steam volume (7b).

7. Humidification module (10, 20, 30) in accordance with claim 4,
**characterized in that** the temperature sensor (5) is arranged inside of the heating device (1).

8. Humidification module (10, 20, 30) in accordance with any of claims 4 to 7,
**characterized in that** the control circuit is implemented to release water into the container (2) via the water inlet (3) when a threshold value of the water vapor temperature measured by the temperature sensor (5) is reached or surpassed.

9. Humidification module (10, 20, 30) in accordance with any of claims 4 to 8,
**characterized in that** the control circuit is implemented to operate the humidification module (10, 20, 30) in a standby mode when it is not required to generate humidity in the form of water vapor, and is further implemented to adjust the heat output of the heating device (1) in the standby mode by means of the control circuit, based on the temperature measured by the temperature sensor (5), such that the temperature remains below the boiling temperature.

10. Incubator or climate chamber with a humidification module (10, 20, 30),
**characterized in that** the humidification module (10, 20, 30) is a humidification module (10, 20, 30) in accordance with any of claims 4 to 9.

## Revendications

1. Procédé de fonctionnement d'un module d'humidification (10, 20, 30), ce module d'humidification (10, 20, 30) ayant :
- un réservoir (2) équipé d'une entrée (3) et d'une sortie de vapeur (4),
- un dispositif de chauffage (1) prévu dans le volume intérieur du réservoir (2), et
- un capteur de température (5) et un circuit de commande pour commander ou réguler l'alimentation en eau par l'entrée d'eau (3) et/ou commander ou réguler la puissance de chauffage du dispositif de chauffage (1),
procédé **caractérisé en ce que**
- le réservoir (2) est rempli avec de l'eau au maximum pour qu'il subsiste un volume de vapeur (7b) dans lequel une partie du dispositif de chauffage (1) dépasse de l'eau et pénètre dans le volume de vapeur (7b) pour surchauffer la vapeur d'eau, et
- le capteur de température (5) est disposé pour mesurer directement ou indirectement la température de la vapeur d'eau, la température mesurée étant utilisée comme paramètre de régulation pour le circuit de commande.

2. Procédé selon la revendication 1,
**caractérisé en ce que**
lorsqu'on atteint ou dépasse le seuil de la température mesurée de la température de la vapeur d'eau mesurée par le capteur de température (5), le circuit de commande laisse arriver de l'eau par l'entrée d'eau (3) dans le réservoir (2).

3. Procédé selon la revendication 1 ou 2,
**caractérisé en ce que**
- le module d'humidification (10, 20, 30) fonctionne en mode d'attente s'il ne doit pas fournir d'humidité sous la forme de vapeur d'eau, et
- la puissance de chauffage de l'élément chauffant (1) en mode d'attente est mesurée par le circuit de commande en utilisant le capteur de température (5) pour réguler la température de façon qu'elle reste en dessous de la température d'ébullition.

4. Module d'humidification (10, 20, 30) pour effectuer le procédé selon l'une des revendications 1 à 3,
comprenant :
- un réservoir (2), ayant une entrée d'eau (3) et une sortie de vapeur (4),
- un dispositif de chauffage (1) installé dans le volume intérieur du réservoir (2) et qui, pour le remplissage maximum du réservoir (2), dépasse en partie de l'eau, un capteur de température (5) et un circuit de commande conçu pour commander ou réguler l'alimentation en eau par l'entrée d'eau (3) et/ou la commande ou la régulation de la puissance de chauffage du dispositif de chauffage (1) en se fondant sur les valeurs mesurées par le capteur de température (5).

5. Module d'humidification (10, 20, 30) selon la revendication 4,
**caractérisé en ce que**
le capteur de température (5) est prévu sur l'enveloppe extérieure du réservoir (2) au-dessus du niveau de remplissage d'eau, maximum, à la hauteur du volume de vapeur (7b).

6. Module d'humidification (10, 20, 30) selon la revendication 4,
**caractérisé en ce que**
le capteur de température (5) est placé dans le volume de vapeur (7b).

7. Module d'humidification (10, 20, 30) selon la revendication 4,
**caractérisé en ce que**
le capteur de température (5) est placé dans le dispositif de chauffage (1).

8. Module d'humidification (10, 20, 30) selon l'une des revendications 4 à 7,
**caractérisé en ce que**
le circuit de commande est conçu pour faire arriver de l'eau par l'entrée d'eau (3) dans le réservoir (2) lorsqu'on atteint ou dépasse un seuil de la température de la vapeur d'eau, mesurée par le capteur de température (5).

9. Module d'humidification (10, 20, 30) selon l'une des revendications 4 à 8,
**caractérisé en ce que**
le circuit de commande est conçu pour le module d'humidification (10, 20, 30) fonctionne dans un mode d'attente s'il n'y a pas à distribuer de l'humidité sous la forme de vapeur d'eau et, en outre, il est conçu pour réguler la puissance de chauffage de l'élément chauffant (1) en mode d'attente par le circuit de commande en utilisant la température mesurée par le capteur de température (5) pour réguler la température pour rester en-dessous de la température d'ébullition.

10. Incubateur ou armoire climatisée comportant un module d'humidification (10, 20, 30),
**caractérisé en ce que**
le module d'humidification (10, 20, 30) est un module d'humidification (10, 20, 30) selon l'une des revendications 4 à 9.
